# EUROPEAN PATENT APPLICATION

(11) **EP 2 702 957 A1**
(43) Date of publication of application: **05.03.2014**
(21) Application number: 12182023.7
(22) Date of filing: 28.08.2012
(51) Int. Cl.: A61B 17/88

(54) **Inflatable bone tamp**

(71) Applicant: Kalotai, Janos, 7556 Ramosch (CH)
(72) Inventor: Kalotai, Janos, 7556 Ramosch (CH)
(74) Representative: Weiss, Wolfgang

(57) **Abstract**

The present invention refers to inflatable bone tamps and devices including same suitable for treating bone and/or joint lesions, particularly bone depressions.

## Description

The present invention refers to inflatable bone tamps and devices including same, suitable for treating bone and/or joint lesions, particularly bone depressions.

The shoulder joint has the greatest range of motion of any joint in the body and as a result is particularly susceptible to dislocation and subluxation. About 1.7% of the total population deal with shoulder dislocations. A dislocated shoulder occurs when the humerus separates from the scapula of the glenohumeral joint. Over 95% of shoulder dislocation cases are anterior-inferior dislocations, that is the humerus separates from the socket in forward-downward direction.

A traumatic luxation is mostly caused by a levering application of force to the stretched extremities. The first luxation often occurs in young adults. After such trauma, recurrent dislocations can occur even when only minor forces are applied, since damages of the capsule-ligament apparatus, detachment of the labrum glenoidale (Bankart lesion) or depressions of the bone heads (e.g. Hill-Sachs lesion) may remain from an initial luxation.

A Hill-Sachs lesion is a bone depression of the humeral head caused by e.g. mechanical impact. By the cadaveric study of Kaar et al. (Am J Sports Med., 2010, 38(3), 594-599), it was found that a Hill-Sachs lesion becomes biomechanically relevant when the lesion has a breadth which corresponds to at least ⅝^{th} of the humeral head's radius.

Usually, dislocated joints are relocated mechanically by known techniques, such as Spaso technique or Cunningham shoulder reduction. In the long run, particularly recurring luxations can only be treated by surgical interventions. It will be understood that post-operational traumata associated therewith have to be minimized in any case.

Minimally invasive surgery is a suitable method to minimize post-operational traumata very effectively due to minimal injury to skin and soft tissue. A swift recovery with few troubles after surgery is assured. In minimally invasive surgery, a cannula is inserted at the position to be operated through a small cut in the skin. Specifically configured surgical instruments and medical devices which can be operated from outside the body are then introduced into the body through the cannula to the position to be operated.

Bone catheters are known in the technical field of minimally invasive surgery for treating bone fractures and symptoms of bone cancer. An expandable balloon is introduced into the cavity of a bone and expanded therein. The deflated balloon is usually guided through the cannula into the bone via a flexible guiding wire, which is removably positioned within the void volume of the balloon catheter. The end of the guiding wire is in direct contact with the inner surface of the expandable balloon, which allows protrusion of the expandable balloon into the bone and subsequent setting of the balloon in the desired position. After the guiding wire has been removed, the expandable balloon may be inflated for compacting cancerous bone marrow against the inner surface of the cortical wall or for stabilizing complicated bone fractures.

One difficulty with this procedure is a proper positioning of the expandable balloon within the bone cavity due to the flexibility of the guiding wires. On the other hand, it is hardly possible to hold the expandable balloon at the desired position over the whole duration of the treatment. It will be understood that particularly in applications where bone and/or joint lesions shall be stabilized or reestablished, it is crucial to locate the balloon at the correct position.

The problem underlying the present invention is the provision of a device for stabilizing bone and/or joint lesions via minimally invasive techniques, which allows an exact placement within a bone cavity and holds a predetermined position during the intervention.

The above problem is solved by the inflatable bone tamps and devices according to the present invention.

Accordingly, in one aspect, the present invention is directed to an inflatable bone tamp, comprising:
(i) an expandable balloon body formed around a central axis defining a hollow channel extending along the central axis, and
(ii) a conduit connected to the expandable balloon body.

The hollow channel formed by the surrounding expandable balloon is preferably open at both axial ends. This configuration allows a free transport of material through the inside of the hollow channel. The hollow channel preferably has a diameter of 1.2-2 mm, more preferably 1.4-1.6 mm. In a preferred embodiment, the expandable balloon is configured such that no material transport between the hollow channel and the expandable balloon body is possible. That is, the material of the balloon forming at least the hollow channel is impermeable to gas, liquids or solid materials.

In a preferred embodiment, the expandable balloon body is adapted to pressures of up to 80 bar, preferably up to 60 bar, even more preferably up to 40 bar. Usually, the lower pressure limit is 2 bar, preferably 4 bar, more preferably 8 bar. The above pressures correspond to the pressures which the expandable balloon body transfers to the surrounding material when maximally expanded. The expandable balloon body of the inflatable bone tamp according to the invention preferably bears a pressure gradient when inflated.

This is particularly obtained by an asymmetric shape of the balloon which allows the exertion of different forces on the bone - at the same pressure inside the balloon.

The material of the expandable balloon body may be any material known in the technical field of balloon catheters. Preferably, the expandable balloon body is made of plastics, such as polyethylene, polypropylene, or polyamide, preferably polyamide and polyethylene.

In order to improve the mechanical stability of the expandable balloon body, it may be reinforced by filaments introduced within the balloon material or surrounding the balloon material. Suitable filaments may be polyamide, carbon or metal fibers.

The expandable balloon body of the inflatable bone tamp according to the invention is not limited to any shape. Preferably, the shape of the expandable balloon bodies matches the cavities into which the balloon bodies are introduced. Preferably, the expandable balloon body is pear-, spherical-, doughnut-, kidney bean-, humpbacked banana-, cylindrical ellipse-, humeral head-, femoral head- or tibial head-shaped.

The expandable balloon body may be coated with a drug or a curable material. When expanding the balloon, particularly only when expanding the balloon, the drug or/and the curable material is transferred to the material forming the cavity into which the expandable balloon body is introduced.

In a preferred embodiment, the drug coating may comprise antimicrobial agents, bone developing agents or anti-cancer agents.

In an alternative embodiment, the expandable balloon body may be coated by a curable material which solidifies after application to the material forming the cavity into which the expandable balloon body is introduced (e.g. bone), thereby strengthening and stabilizing the material. Curing may be initiated by chemical agents or by irradiation. Preferred curable materials may be resins, such as two-component resins, preferably polyurethane resins, or hydraulically binding materials, such as organic or inorganic cements or ceramics.

Further, the inflatable bone tamp comprises a conduit which is connected to the expandable balloon body. The conduit is connected to the expandable balloon body such that a medium, preferably a fluid such as a liquid or a gas, can be introduced into the balloon body for expansion and removed from the balloon body for deflation. The conduit is preferably attached to the back part of the expandable balloon body.

In a preferred embodiment, the end of the conduit which is not connected to the balloon may be connected with expanding means, such as a pump. The expanding means are configured such that the expandable balloon body is expanded to the required pressures. The expanding means may be part of the inflatable bone tamp.

In another aspect, the present invention relates to a device or kit for stabilizing bone lesions, comprising
(i) a guiding wire,
(ii) an inflatable bone tamp as described above,
(iii) a cannula, and
(iv) optionally a curette.

In a preferred embodiment the present invention relates to a device or kit for stabilizing bone lesions, comprising
(i) a guiding wire,
(ii) an inflatable bone tamp as described above, and
(iii) a cannula.

In a preferred embodiment, the guiding wire is substantially rigid. In contrast to the commonly used guiding wires in the technical field of e.g. balloon catheters, the more rigid guiding wire of the invention allows an exact placement and positioning of the inflatable bone tamp.

The guiding wire preferably has a diameter of 1.2-2 mm, preferably 1.4-1.6 mm. The guiding wire is preferably made from titanium, stainless steel, nickel alloys and combinations thereof. In a preferred embodiment, the guiding wire has a sharp or threaded pin, configured such that it can be introduced into bone material by rotation, e.g. by hand or by using a drill.

The cannula (a hollow cylinder) preferably has a diameter of 3-15 mm, more preferably 3-10 mm, even more preferably 5-8 mm. The cannula may further comprise sharpened edges or a removable sharpened head, such as a drilling head, at one end. Preferably, the sharpened head has a concentrical opening, which is preferably adapted to the diameter of the guiding wire. Above described cannulae having sharpened edges or a removable sharpened head are also known as trocar. In a preferred embodiment, the cannula, more preferably the trocar, may be used to create an access to the position of the body to be treated.

A curette is a surgical instrument designed for scraping biological material, such as bone marrow, medulla ossium, spongy bone, etc. A curette may be used to form a void volume within a body part. Any curette known in the art may be used according to the invention.

In a preferred embodiment, the expandable balloon body in its deflated condition is folded around the hollow channel. This arrangement ensures a minimized volume.

The guiding wire of the device according to the invention is preferably positioned slidingly within the hollow channel extending along the central axis of the expandable balloon body as described above.

In a preferred embodiment, the inflatable bone tamp and the guiding wire are arranged in a way, such that the conduit connected to the expandable balloon body and the end of the guiding wire to be inserted into the body are each arranged in an opposite direction.

In a preferred embodiment, the guiding wire and the expandable balloon body in its deflated condition are positioned within the cannula.

In a most preferred embodiment, the expandable balloon body is folded in its deflated condition around the hollow channel, which bears the guiding wire. Said assembly is preferably arranged within a cannula.

In another aspect, the present invention refers to a method for stabilizing bone and/or joint lesions comprising the steps of
(i) inserting a guiding wire through a bone having a cavity,
(ii) fixing the guiding wire at the bone outlet section,
(iii) introducing a cannula guided by the guiding wire into the bone cavity,
(iv) placing the expandable balloon body of an inflatable bone tamp as described above into the bone cavity,
(v) optionally fixing the guiding wire at the bone inlet section, and
(vi) inflating the expandable balloon body of the inflatable bone tamp.

In step (i), a guiding wire as described above is inserted through a bone having a cavity. Usually bones exhibit a mineralized osseous tissue, also called bone tissue, that gives it rigidity, and a coral-like three-dimensional internal structure also including marrow, endosteum, periosteum, nerves, blood vessels, cartilage and optionally bruised material. According to the present invention "cavity" as used herein means the overall internal structure surrounded by the osseous tissue.

The guiding wire may be introduced into the bone by rotation, e.g. by hand, by using a hand drill or by using a drilling machine. The area around the channel formed into the bone material by the guiding wire (i.e. from the bone surface to the inner cavity surface) defines the bone inlet section. The area around the channel formed by the guiding wire into the bone material - after the guiding wire has passed the bone cavity (i.e. from the inner cavity surface to the bone surface) - defines the bone outlet section. In a preferred embodiment, the bone outlet section is located on the other side of the bone cavity opposite of the bone inlet section. The exact positioning of the guiding wire may be affected by radiological control, such as X-ray imaging or other methods known in the art.

In step (ii) the guiding wire is fixed at the bone outlet section. That is, the guiding wire may be fixed on the surface of the bone at the outlet section, e.g. by a staple, clamp. In an alternative, the guiding wire may be anchored into the bone material at the bone outlet section, e.g. by a thread or a hook. The fixation of the guiding wire allows a tightening or extending the guiding wire from outside the body, thereby allowing an exact placement of any devices to be guided to the desired position.

A cannula as described above is introduced into the bone cavity in step (iii). The cannula preferably encompasses the guiding wire, which allows an optimal guidance by the guiding wire. If a cannula is used having a sharpened head with a concentrical opening, the cannula may be guided by the opening along the guiding wire into the bone cavity. The guiding wire thus allows an exact positioning of the cannula in the bone cavity.

In order to obtain optimal positioning of the cannula, the guiding wire may be tightened from outside the body while the cannula is introduced into the bone cavity. If present, the cannula's drilling head may be removed after the cannula has been placed into the bone cavity. The cannula establishes an access to the position to be treated.

If necessary, a curette may be used in order to at least partially remove the coral-like three-dimensional internal structure within the bone cavity. Thereby, a void volume is formed within the bone cavity which facilitates introduction of the inflatable bone tamp. In a preferred embodiment, the curette is forced through the cannula, preferably guided along the guiding wire.

In the next step, the expandable balloon body of an inflatable bone tamp as described above is placed into the bone cavity (step (iv)). Preferably, the expandable balloon of the inflatable bone tamp as described above is protruded to the desired position along the guiding wire, which is preferably tightened. Preferably, the expandable balloon of the inflatable bone tamp is slidingly placed around the guiding wire, so that the guiding wire extends along the hollow channel of the expandable balloon body.

In a preferred embodiment, the conduit of the inflatable bone tamp is positioned backward in order to assure easy access to the expandable balloon body from outside the human or animal body.

Preferably, the deflated balloon body bearing the guiding wire within the hollow channel is forced through the cannula to the desired position.

Due to the possibility of the exact placement of the guiding wire, the method according to the present invention allows an exact positioning of the expandable balloon of the inflatable bone tamp.

After the expandable balloon body has been exactly positioned into the bone cavity, the guiding wire may be tightened and fixed at the bone inlet section.

The guiding wire may preferably be fixed on the surface of the bone at the inlet section, e.g. by a staple or clamp. In an alternative, the guiding wire may be anchored into the bone material at the bone inlet section, e.g. by a thread, or a hook. In an alternative embodiment, the guiding wire may be preferably fixed at a tightened condition outside the body by holding the guiding wire under constant tension.

Such fixation assures that the balloon is held in the desired position and does not dislocate during the dilatation process.

In the next step, the expandable balloon body of the inflatable bone tamp is inflated via the conduit. The conduit preferably extends through the cannula. The conduit is preferably connected to expanding means as described above at its backward end. The balloon can be expanded by pressing a medium, e.g. a gas or a liquid, into the expandable balloon body.

In a preferred embodiment, the expandable balloon body is inflated to pressures of up to 80 bar, preferably up to 60 bar, even more preferably up to 40 bar. Usually, the lower pressure limit is 2 bar, preferably 4 bar, more preferably 8 bar. The above pressures correspond to the pressures which the expandable balloon body exerts to the surrounding bone material when maximally expanded.

By the pressure exerted to the bone from inside the bone, bone and/or joint lesions can be stabilized or bone depressions can be restored (so-called inside-out repair). In a preferred embodiment, bone depressions are restored. The bone and/or joint lesions are particularly selected from humeral head lesions, femoral head lesions, tibial head lesions, calcaneus lesions, or distal tibia lesions.

The method of the present invention is particularly suitable for treating Hill-Sachs lesions, femoral or tibial head lesions, particularly Hill-Sachslesions.

The tight anchorage of the guiding wire allows an exact positioning of the inflatable bone tamp according to the invention. Moreover, the method according to the invention assures that no dislocation of the expandable balloon body occurs during dilatation. The present method thus leads to excellent surgical results, which up to now have not been possible by using the common methods.

After the bone and/or joint lesions have been restored, the expandable balloon body may be deflated and removed from the bone through the cannula. Also, the guiding wire may be detached and removed through the cannula. In a preferred embodiment, the cavity caused by the method according to the invention may then be filled with a medium, which may be introduced via the cannula. Exemplary media for filling the cavity may be inorganic or organic cements, e.g. calcium phosphate containing cements.This procedure may further stabilize bone and/or joint lesions, thereby avoiding e.g. recurrent lesions.

In a final step, the cannula may be removed.

The present invention is further exemplified by the following Figures.

| | | |
|---|---|---|
| **Figure 1** | A | Inflatable bone tamp according to the invention (cross-section) |
| | B | Inflatable bone tamp according to the invention (top-view) |
| **Figure 2** | A | Bone having introduced a guiding wire |
| | B | Bone having introduced a cannula |
| | C | Bone having introduced an expandable balloon body |

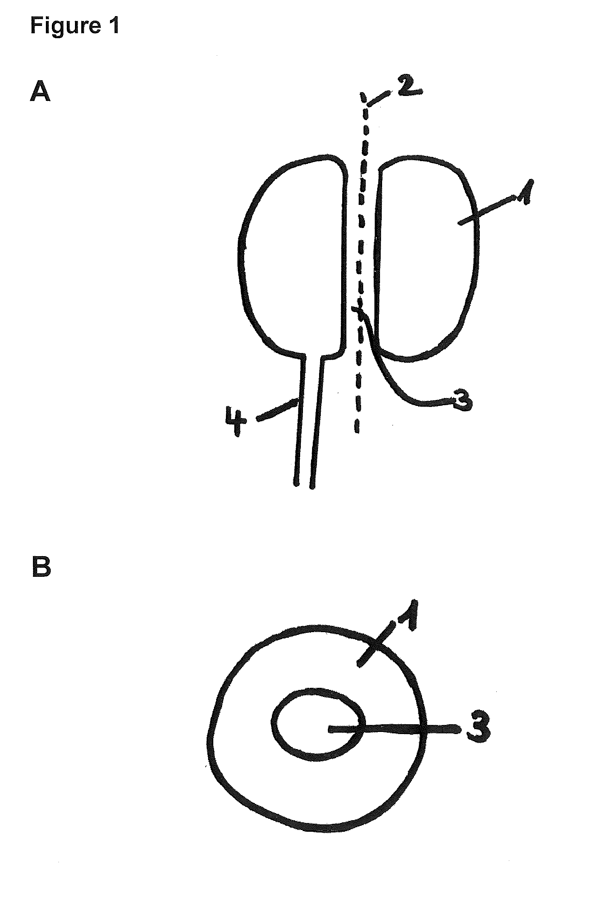

Figure 1 shows an expandable balloon body of an inflatable bone tamp. The expandable balloon body (1) is formed around a central axis (2) defining a hollow channel (3) extending along the central axis. The conduit (4) is connected to the expandable balloon body (1).

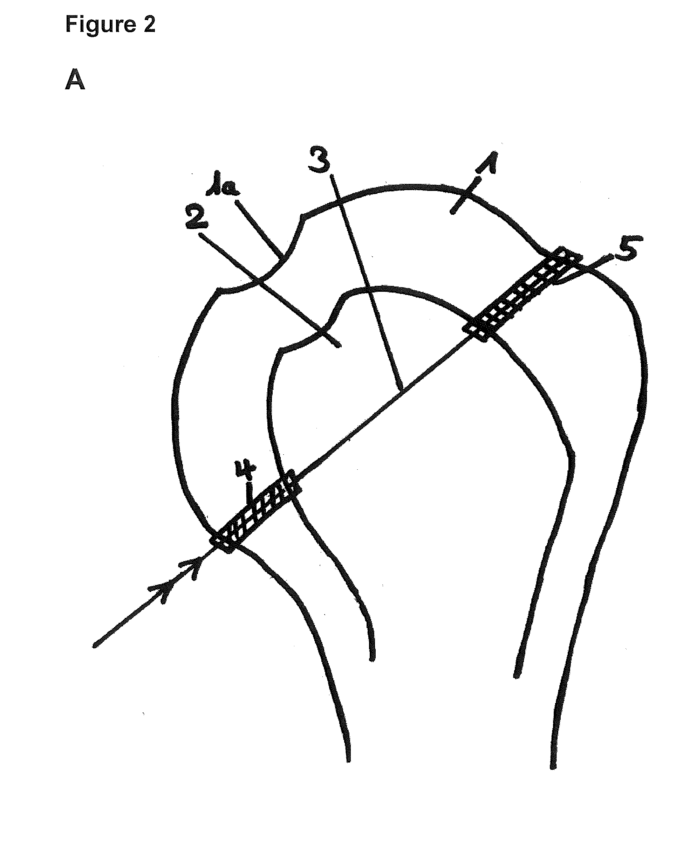

Figure 2A shows a bone (1) having a cavity (2) and including a bone depression (1a). A guiding wire (3) is inserted through the bone cavity (2). The guiding wire is introduced into the bone at the bone inlet section (hatched) (4) and extrudes at the bone outlet section (hatched) (5). The guiding wire may be fixed at the bone outlet section (5).

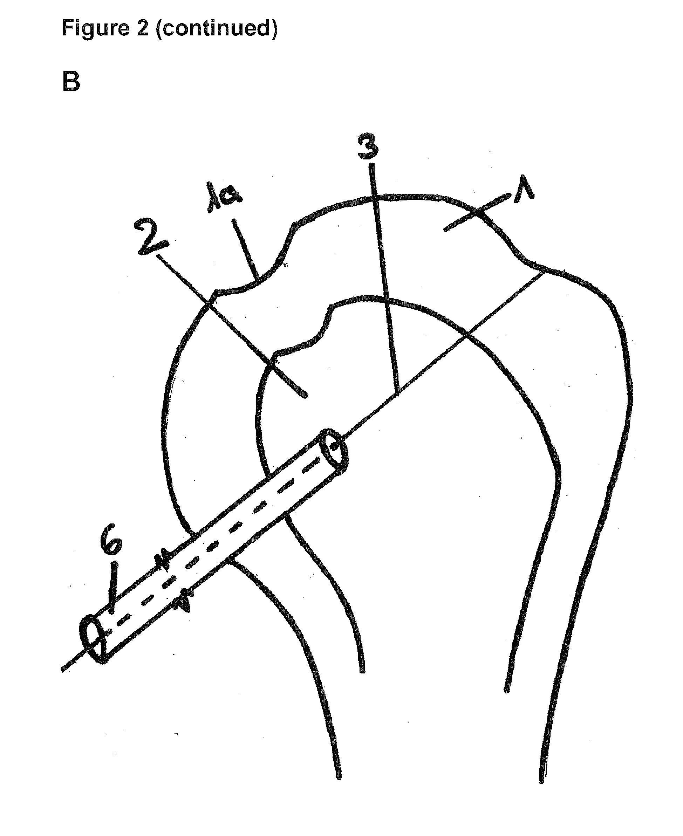

In Figure 2B, a cannula (6) is introduced into the bone cavity, wherein the cannula has been guided via the guiding wire into the bone cavity. In this preferred embodiment, the cannula (6) surrounds the guiding wire (3).

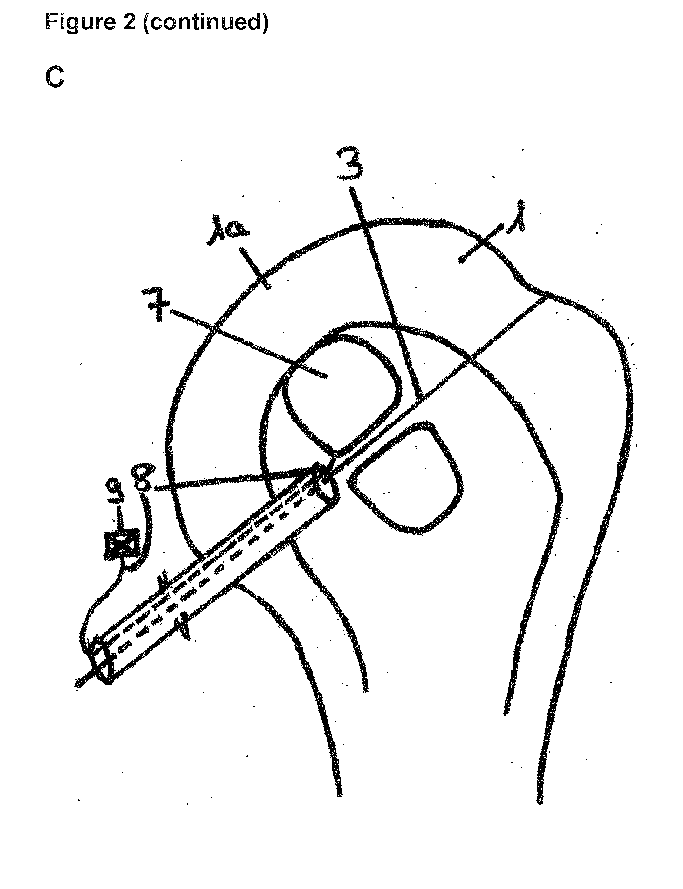

In Figure 2C, the expandable balloon body (7) has been forced along the guiding wire (3), which is positioned within the hollow channel of the expandable balloon body, into the bone cavity. The expandable balloon body is shown in its expanded condition. The conduit (8) of the inflatable bone tamp according to the invention is connected forwardly with the expandable balloon body and backwardly with a expanding means (9). The conduit extends through the cannula. Due to the possibility of exact positioning of the expandable balloon within the bone cavity, the bone depression (former 1 a) is restored after having expanded the expandable balloon body.

The present invention includes the following items:
1. Inflatable bone tamp, comprising:
   (i) an expandable balloon body formed around a central axis defining a hollow channel extending along the central axis, and
   (ii) a conduit connected to the expandable balloon body.
2. The inflatable bone tamp according to item 1, wherein both axially ends of the hollow channel are open.
3. The inflatable bone tamp according to any of items 1-2, wherein the channel has a diameter of 1.2-2 mm, preferably 1.4-1.6 mm.
4. The inflatable bone tamp according to any of items 1-3, wherein the expandable balloon body is adapted to pressures of up to 80 bar, preferably up to 60 bar.
5. The inflatable bone tamp according to any of items 1-4, wherein the expandable balloon body bears a pressure gradient when inflated.
6. The inflatable bone tamp according to any of items 1-5, wherein the expandable balloon body is reinforced by filaments, such as polyamide or carbon fibers.
7. The inflatable bone tamp according to any of items 1-6, wherein the expandable balloon body is pear-, spherical-, doughnut-, kidney bean-, humpbacked banana-, cylindrical ellipse-, humeral head-, femoral head- or tibial head-shaped.
8. The inflatable bone tamp according to any of items 1-7, wherein the expandable balloon body is coated with a drug or a curable material.
9. The inflatable bone tamp according to any of items 1-8, wherein the conduit connects the expandable balloon body with expanding means, such as a pump.
10. Device for stabilizing bone lesions, comprising
   (i) a guiding-wire,
   (ii) an inflatable bone tamp according to any of items 1-9,
   (iii) a cannula, and
   (iv) optionally a curette.
11. The device according to item 10, wherein the guiding wire is substantially rigid.
12. The device according to any of items 10-11, wherein the guiding wire has a diameter of 1.2-2 mm, preferably 1.4-1.6 mm.
13.The device according to any of items 10-12, wherein the guiding wire is made from titanium, stainless steel, nickel alloy and combinations thereof.
14. The device according to any of items 10-13, wherein the guiding wire has a threaded or sharp pin.
15. The device according to any of items 10-14, wherein the guiding wire is slidingly positioned within the hollow channel extending along the central axis of the expandable balloon body.
16.The device according to any of items 10-15, wherein the guiding wire and the expandable balloon body in its deflated condition are positioned within the cannula.
17. The device according to any of items 10-16, wherein the cannula has a diameter of 3-10 mm, preferably 5-8 mm.
18. Method for stabilizing bone and/or joint lesions comprising the steps of
   (i) inserting a guiding wire through a bone having a cavity,
   (ii) fixing the guiding wire at the bone outlet section,
   (iii) introducing a cannula guided by the guiding wire into the bone cavity,
   (iv) placing the expandable balloon body of an inflatable bone tamp as described above into the bone cavity,
   (v) optionally fixing the guiding wire at the bone inlet section, and
   (vi) inflating the expandable balloon body of the inflatable bone tamp.
19. The method according to item 18, wherein the bone and/or joint lesion is a humeral head lesion, a femoral head lesion, a tibial head lesion, a calcaneus lesion or a distal tibia lesion.
20. The method according to any of items 18-19, wherein the bone lesion is a Hill-Sachs lesion.
21. the method according to any of items 18-20, wherein the guiding wire is fixed via a clamp or a thread at the bone outlet section.
22. The method according to any of items 18-21, wherein the expandable balloon body of the inflatable bone tamp is guided to the desired position along the guiding wire.
23. The method according to item 22, wherein the deflated balloon body bearing the guiding wire within the hollow channel is forced through the cannula to the desired position.
24. The method according to any of items 18-23, wherein the guiding wire is fixed via a clamp or a thread at the bone inlet section or is fixed outside the body.
25. The method according to any of items 18-24, wherein the expandable balloon body is inflated up to a pressure of 80 bar, preferably 60 bar.

## Claims

1. Inflatable bone tamp, comprising:
(i) an expandable balloon body formed around a central axis defining a hollow channel extending along the central axis, and
(ii) a conduit connected to the expandable balloon body.

2. The inflatable bone tamp according to claim 1, wherein the channel has a diameter of 1.2-2 mm, preferably 1.4-1.6 mm.

3. The inflatable bone tamp according to any of claims 1-2, wherein the expandable balloon body is adapted to pressures of up to 80 bar, preferably up to 60 bar.

4. The inflatable bone tamp according to any of claims 1-3, wherein the expandable balloon body is pear-, spherical-, doughnut-, kidney bean-, humpbacked banana-, cylindrical ellipse-, humeral head-, femoral head- or tibial head-shaped.

5. The inflatable bone tamp according to any of claims 1-4, wherein the conduit connects the expandable balloon body with expanding means, such as a pump.

6. Device for stabilizing bone lesions, comprising
(i) a guiding-wire,
(ii) an inflatable bone tamp according to any of claims 1-5, and
(iii) a cannula.

7. The device according to claim 6, wherein the guiding wire is substantially rigid.

8. The device according to any of claims 6-7, wherein the guiding wire has a diameter of 1.2-2 mm, preferably 1.4-1.6 mm.

9. The device according to any of claims 6-8, wherein the guiding wire is slidingly positioned within the hollow channel extending along the central axis of the expandable balloon body.

10. The device according to any of claims 6-9, wherein the cannula has a diameter of 3-10 mm, preferably 5-8 mm.

11. Method for stabilizing bone and/or joint lesions comprising the steps of
(i) inserting a guiding wire through a bone having a cavity,
(ii) fixing the guiding wire at the bone outlet section,
(iii) introducing a cannula guided by the guiding wire into the bone cavity,
(iv) placing the expandable balloon body of an inflatable bone tamp as claimed in any of claims 1-10 into the bone cavity,
(v) optionally fixing the guiding wire at the bone inlet section, and
(vi) inflating the expandable balloon body of the inflatable bone tamp.

12. The method according to claim 11, wherein the bone and/or joint lesion is a humeral head lesion, a femoral head lesion, a tibial head lesion, a calcaneus lesion or a distal tibia lesion, particularly a Hill-Sachs lesion.

13. The method according to any of claims 11-12, wherein the guiding wire is fixed via a clamp or a thread at the bone outlet section.

14. The method according to any of claims 11-13, wherein the deflated balloon body bearing the guiding wire within the hollow channel is forced through the cannula to the desired position.

15. The method according to any of claims 11-14, wherein the expandable balloon body is inflated up to a pressure of 80 bar, preferably 60 bar.
